# EUROPÄISCHE PATENTANMELDUNG

(11) **EP 3 662 883 A1**
(43) Veröffentlichungstag der Anmeldung: **10.06.2020**
(21) Anmeldenummer: 19208779.9
(22) Anmeldetag: 13.11.2019
(51) Int. Cl.: A61K 8/02, A61K 8/36, A61K 8/44, A61Q 5/02, A61Q 19/10

(54) **BEI RAUMTEMPERATUR FESTES NATÜRLICHES KOSMETISCHES REINIGUNGSMITTEL**

(30) Priorität: 05.12.2018 DE 102018221038
(71) Anmelder: Henkel AG & Co. KGaA, 40589 Düsseldorf (DE)
(72) Erfinder: Schelges, Heike, 47877 Willich (DE); Scholz, Elvira, 40764 Langenfeld (DE)

(57) **Zusammenfassung**

Die vorliegende Erfindung betrifft bei 25°C feste kosmetische Reinigungsmittel, enthaltend -jeweils bezogen auf ihr Gewicht - 5 bis 90 Gew.-% Alkalisalze von Fettsäuren (Seifen); 2 bis 50 Gew.-% Aclyglutamat(e) sowie 0,1 bis 10 Gew.-% Betain (*N,N,N-*Trimethylammonioacetat bzw. *N*,*N*,*N*-Trimethylglycin).

## Beschreibung

Die vorliegende Erfindung betrifft natürliche feste Reinigungs- und Konditioniermittel für Haut und insbesondere Haar, welche den gesteigerten Verbraucherwunsch nach ökologisch akzeptablen und natürlichen Produkten befriedigen.

Es besteht derzeit ein erhöhtes Verbraucherinteresse an kosmetischen Produkten, die ein Naturkosmetik-Label tragen und frei von diskutierten Inhaltstoffen sind. Um dem Verbraucher die Sicherheit zu geben, ein ökologisch einwandfreies und sicher natürliches Produkt zu erwerben, gibt es etliche Zertifizierungsgesellschaften, die nach Prüfung des Einzelfalles entsprechender Siegel vergeben.
Bekannt ist hier unter anderem das BDIH-Label als Erkennungszeichen für Produkte, die den strengen Anforderungen an echte Naturkosmetik gerecht werden. Aktuell verfügen rund 200 Lizenznehmer mit über 300 Marken über das BDIH-Label (Bundesverband der Industrie- und Handelsunternehmen für Arzneimittel, Reformwaren, Nahrungsergänzungs- und kosmetische Mittel, kurz BDIH). Etwa 40 befinden sich im europäischen Ausland und weitere 40 außerhalb Europas. In Summe gibt es derzeit rund 9000 Produkte, die mit dem BDIH-Siegel deklariert sind. Ergänzend zum BDIH-Siegel für kontrollierte Naturkosmetik wurden weitere Siegel, beispielsweise das französische "Ecocert"-Label etabliert. "Organic" steht für die gleichen Inhaltsstoffanforderungen wie bei einem weiteren, dem "NaTrue- Siegel". Gleiches gilt für "Made with organic ingredients". Auf amerikanischen Produkten gibt es das "USDA-Siegel", in England zusätzlich das Label "Soil Association".
Seit 2010 wurde ein weiteres Siegel mit Namen "Cosmos" für den Bereich der Natur- und Biokosmetik etabliert. Das neue Label ist bindend für sämtliche Neuentwicklungen von Natur- und Biokosmetik. Es gilt für sämtliche Mitglieder von BDIH, Cosmebio, ICEA, Ecocert und Soil Association. Sie repräsentieren im Kollektiv das weltweit größte System zur Zertifizierung von Bio-Beautyprodukten. Seit dieser Zeit werden Produkte der Mitglieder, gemäß internationalem Cosmos-Standard AISBL, zertifiziert. Mehrere tausend Rohstoffe und auch Produkte haben in dieser Zeit bereits die Anforderungsprüfungen erfolgreich bestanden. Alle Erzeugnisse, die nach dem 1.1.2017 als Natur- oder Biokosmetik angemeldet und geprüft werden, erhalten dann die Cosmos- Zertifizierung der jeweiligen Mitgliederorganisation. So gibt es beispielsweise Cosmos-BDIH oder Cosmos- Ecocert-Label.
Unterschieden wird in die Kontrolle von Naturkosmetik sowie in die Zertifizierung von Bio-Produkten. Letztere muss zusätzlich geforderte Anteile von Inhaltsstoffen aus biologischem Anbau nachweisen. So müssen bei einem Bio-Kosmetikprodukt mindestens 95 Prozent der gewonnenen pflanzlichen Bestandteile aus ökologischer Landwirtschaft stammen.

0arüber hinaus geht der Trend zu Produkten, die auf eine Plastikumverpackung verzichten können. Hier bieten sich wasserarme Systeme wie Seifen und Syndets an. Um gleichzeitig eine gute Perfomance an Haut und Haar bei gleichzeitiger Mildheit zu garantieren, stehen nur wenige schäumende Tenside in Pulverform zur Verfügung, die auch dem Cosmos-Standard entsprechen.

Es besteht daher ein Bedarf an kosmetischen Reinigungs- und Konditioniermitteln für Haut und insbesondere Haar, die Cosmos-zertifizierbar sind und mit einem deutlich reduzierten Verpackungsaufwand vertrieben werden können.

Der vorliegenden Erfindung lag die Aufgabe zugrunde, die vorstehenden genannten Probleme zu lösen. Die der Erfindung zugrundeliegende Aufgabe wird gelöst durch den Gegenstand von Anspruch 1. Ein erster Gegenstand der Erfindung ist ein bei 25°C festes kosmetisches Reinigungsmittel, enthaltend -jeweils bezogen auf sein Gewicht -
a) 5 bis 90 Gew.-% Alkalisalze von Fettsäuren (Seifen);
b) 2 bis 50 Gew.-% Aclyglutamat(e);
c) 0,1 bis 10 Gew.-% Betain (*N,N,N*-Trimethylammonioacetat bzw. *N,N,N*-Trimethylglycin).

Die erfindungsgemäßen Reinigungsmittel sind bei 25°C fest. Feste Stoffe im Sinne der vorliegenden Anmeldung sind dreidimensionale formstabile Gebilde, die nicht flüssig oder gasförmig sind, d.h. ihre äußere Form auch ohne ein sie umgebendes Gefäß aufrechterhalten. Über Dichte oder Elastizität bzw. weitere physikalische Eigenschaften sagt der Begriff "fest" hingegen nichts aus, so dass auch Gallerten, Sülzen, Buttern usw. fest im Sinne der Erfindung sein können, solange sie bei 25°C formstabil sind.

Die erfindungsgemäßen Reinigungsmittel enthalten als ersten Inhaltsstoff eine oder mehrere Seife(n) in einer Gesamtmenge aller Alkalisaze von fettsäuren von 5 bis 90 Gew.-%. Mit besonderem Vorzug wird/werden die Seife(n) innerhalb engerer Mengenbereiche eingesetzt. Hier sind erfindungsgemäß bevorzugte kosmetische Reinigungsmittel dadurch gekennzeichnet, dass sie 10 bis 89 Gew.-%, vorzugsweise 20 bis 88 Gew.-%, weiter bevorzugt 30 bis 87 Gew.-%, noch weiter bevorzugt 40 bis 86 Gew.-% und insbesondere 50 bis 85 Gew.-% Alkalisalze von Fettsäuren (Seifen) enthalten.

Fettsäuren sind aliphatische Carbonsäuren der Formel **R¹CO-OH**, in der R¹CO für einen aliphatischen, linearen oder verzweigten Acylrest mit 6 bis 22 Kohlenstoffatomen und 0 und/oder 1, 2 oder 3 Doppelbindungen steht. Typische Beispiele sind Capronsäure, Caprylsäure, 2-Ethylhexansäure, Caprinsäure, Laurinsäure, Isotridecansäure, Myristinsäure, Palmitinsäure, Palmitoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z.B. bei der Druckspaltung von natürlichen Fetten und Ölen anfallen. Bevorzugt sind technische Fettsäuren mit 12 bis 18 Kohlenstoffatomen, wie beispielsweise Kokos-, Palm-, Palmkern- oder Talgfettsäure, wobei im Hinblick auf die vorliegende Erfindung der Einsatz von Kokosfettsäuren besonders bevorzugt ist und auf den Einsatz von Rohstoffen aus Palmöl verzichtet werden sollte.

Besonders bevorzugte erfindungsgemäße Reinigungsmittel sind frei von Seifen, welche aus Palmöl hergestellt wurden.

Unter den Alkalisalzen sind die Natriumsalze wegen ihrer anwendungstechnischen Eigenschaften im Rahmen der vorliegenden Erfindung besonders bevorzugt. Besonders bevorzugte erfindungsgemäße kosmetische Reinigungsmittel enthalten 10 bis 89 Gew.-%, vorzugsweise 20 bis 88 Gew.-%, weiter bevorzugt 30 bis 87 Gew.-%, noch weiter bevorzugt 40 bis 86 Gew.-% und insbesondere 50 bis 85 Gew.-% Natriumsalze von C₁₂₋₁₈-Fettsäuren.

Die erfindungsgemäßen Reinigungsmittel enthalten als zweiten Inhaltsstoff 2 bis 50 Gew.-% Aclyglutamat(e). Diese Verbindungen werden aus L-Glutaminsäure und in der Natur vorkommenden Fettsäuren hergestellt und zeichnen sich durch geringes Schaumbildungs- und gutes Waschvermögen aus.

Mit besonderem Vorzug enthalten die erfindungsgemäßen kosmetische Reinigungsmittel 3 bis 40 Gew.-%, vorzugsweise 4 bis 35 Gew.-%, weiter bevorzugt 5 bis 30 Gew.-%, noch weiter bevorzugt 6 bis 25 Gew.-% und insbesondere 7,5 bis 15 Gew.-% Acylglutamate der Formel **(I)** 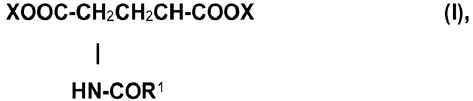 in der R¹CO für einen linearen oder verzweigten Acylrest mit 6 bis 22 Kohlenstoffatomen und 0 und/oder 1, 2 oder 3 Doppelbindungen und X für Wasserstoff, ein Alkali- und/oder Erdalkalimetall-, Ammonium-, Alkylammonium-, Alkanolammonium- oder Glucammonium- steht.

Wiederum im Hinblick auf die Zertifizierungsmöglichkeit als Naturkosmetik ist es im Rahmen der vorliegenden Erfindung bevorzugt, als Kation X Alkalimetallionen und insbesondere Natriumionen einzusetzen, Bei der Fettresten ist wie bei den Seifen der Einsatz von Kokosfettsäuren besonders bevorzugt, und es sollte auf den Einsatz von Rohstoffen aus Palmöl verzichtet werden.

Besonders bevorzugte erfindungsgemäße Reinigungsmittel sind frei von Acylglutamaten, welche aus Palmöl hergestellt wurden.

Ganz besonders bevorzugte erfindungsgemäße kosmetische Reinigungsmittel sind dadurch gekennzeichnet, dass sie ein Acylglutamat enthalten, in dem X für Na und R¹CO für einen Acylrest steht, der von Kokosöl abgeleitet ist (INCI: Sodium Cocoyl Glutamate).

Die erfindungsgemäßen Mittel können zusätzlich zu der bzw. den Seife(n) und dem bzw. den Acylglutamat(en) weitere Tenside enthalten. Im Hinblick auf die eingangs erwähnte Problematik und die Hautfreundlichkeit der Mittel hat es sich als vorteilhaft erwiesen, wenn nur Tenside aus bestimmten Stoffgruppen eingesetzt werden. Als besonders vorteilhaft im Hinblick auf die Verbraucherakzeptanz, die Hautfreundlichkeit und die Anwendungeigenschaften haben sich erfindungsgemäße kosmetische Reinigungsmittel erwiesen, die ausschließlich Tensid(e) aus den Gruppen der
- Alkyloligo- und -polysaccharide
- Betaine
enthalten.

Es ist insbesondere bevorzugt, wenn die erfindungsgemäßen kosmetischen Reinigungsmittel - bezogen auf ihr Gewicht - weniger als 1 Gew.-%, vorzugsweise weniger als 0,5 Gew.-%, weiter bevorzugt weniger als 0,5 Gew.-% und insbesondere weniger als 0,1 Gew.-% Sulfat-haltige(s) Tensid(e) enthalten, wobei äußerst bevorzugte Mittel vollständig frei sind von sulfathaltigen Tensiden.

Die optional mit Vorzug einsetzbaren Tenside werden nachstehend beschrieben.
Alkyl- und Alkenyloligoglykoside stellen bekannte nichtionische Tenside dar, die sich durch die Formel

**R¹O-[G]**ₚ

beschreiben lassen, in der R¹ für einen Alkyl- und/oder Alkenylrest mit 4 bis 22 Kohlenstoffatomen, G für einen Zuckerrest mit 5 oder 6 Kohlenstoffatomen und p für Zahlen von 1 bis 10 steht. Sie können nach den einschlägigen Verfahren der präparativen organischen Chemie erhalten werden.

Die Alkyl- und/oder Alkenyloligoglykoside können sich von Aldosen bzw. Ketosen mit 5 oder 6 Kohlen-stoffatomen, vorzugsweise der Glucose ableiten. Die bevorzugten Alkyl- und/oder Alkenyloligoglykoside sind somit Alkyl- und/oder Alkenyloligo**glucoside**. Die Indexzahl p in der allgemeinen Formel gibt den Oligomerisierungsgrad (DP), d. h. die Verteilung von Mono- und Oligoglykosiden an und steht für eine Zahl zwischen 1 und 10. Während p in einer gegebenen Verbindung stets ganzzahlig sein muß und hier vor allem die Werte p = 1 bis 6 annehmen kann, ist der Wert p für ein bestimmtes Alkyloligo-glykosid eine analytisch ermittelte rechnerische Größe, die meistens eine gebrochene Zahl darstellt. Vorzugsweise werden Alkyl- und/oder Alkenyloligoglykoside mit einem mittleren Oligomerisierungsgrad p von 1,1 bis 3,0 eingesetzt. Aus anwendungstechnischer Sicht sind solche Alkyl- und/oder Alkenyloligoglykoside bevorzugt, deren Oligomerisierungsgrad kleiner als 1,7 ist und insbesondere zwischen 1,2 und 1,4 liegt. Der Alkyl- bzw. Alkenylrest R¹ kann sich von primären Alkoholen mit 4 bis 11, vorzugsweise 8 bis 10 Kohlenstoffatomen ableiten. Typische Beispiele sind Butanol, Capronalkohol, Caprylalkohol, Caprinalkohol und Undecylalkohol sowie deren technische Mischungen, wie sie beispielsweise bei der Hydrierung von technischen Fettsäuremethylestern oder im Verlauf der Hydrierung von Aldehyden aus der Roelen'schen Oxosynthese erhalten werden. Bevorzugt sind Alkyloligoglucoside der Kettenlänge C₈-C₁₀ (DP = 1 bis 3), die als Vorlauf bei der destillativen Auftrennung von technischem C₈-C₁₈-Kokosfettalkohol anfallen und mit einem Anteil von weniger als 6 Gew.-% C₁₂-Alkohol verunreinigt sein können sowie Alkyloligoglucoside auf Basis technischer C_{9/11}-Oxoalkohole (DP = 1 bis 3). Der Alkyl- bzw. Alkenylrest R¹ kann sich ferner auch von primären Alkoholen mit 12 bis 22, vorzugsweise 12 bis 14 Kohlenstoffatomen ableiten. Typische Beispiele sind Laurylalkohol, My-ristylalkohol, Cetylalkohol, Palmoleylalkohol, Stearylalkohol, Isostearylalkohol, Oleylalkohol, Elaidyl-alkohol, Petroselinylalkohol, Arachylalkohol, Gadoleylalkohol, Behenylalkohol, Erucylalkohol, Brassidylalkohol sowie deren technische Gemische, die wie oben beschrieben erhalten werden kön-nen. Bevorzugt sind Alkyloligoglucoside auf Basis von gehärtetem C_{12/14}-Kokosalkohol mit einem DP von 1 bis 3.

Bevorzugte erfindungsgemäße Reinigungsmittel sind dadurch gekennzeichnet, dass sie 0,1 bis 10 Gew.-%, vorzugsweise 0,25 bis 7,5 Gew.-%, weiter bevorzugt 0,5 bis 5 Gew.-%, noch weiter bevorzugt 0,75 bis 2,5 Gew.-% und insbesondere 1 bis 2 Gew.-% Tensid(e) aus der Gruppe der Alkyloligo- und -polysaccharide enthalten.

Betaine stellen bekannte Tenside dar, die überwiegend durch Carboxyalkylierung, vorzugsweise Carb-oxymethylierung von aminischen Verbindungen hergestellt werden. Vorzugsweise werden die Aus-gangsstoffe mit Halogencarbonsäuren oder deren Salzen, insbesondere mit Natriumchloracetat kon-densiert, wobei pro Mol Betain ein Mol Salz gebildet wird. Ferner ist auch die Anlagerung von unge-sättigten Carbonsäuren, wie beispielsweise Acrylsäure möglich. Beispiele für geeignete Betaine stellen die Carboxyalkylierungsprodukte von sekundären und insbesondere tertiären Aminen dar, die sich durch die Formel 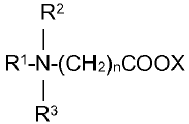 beschreiben lassen, in der R¹ für Alkyl- und/oder Alkenylreste mit 6 bis 22 Kohlenstoffatomen, R² für Wasserstoff oder Alkylreste mit 1 bis 4 Kohlenstoffatomen, R³ für Alkylreste mit 1 bis 4 Kohlenstoffatomen, n für Zahlen von 1 bis 6 und X für ein Alkali- und/oder Erdalkalimetall oder Ammonium steht. Typische Beispiele sind die Carboxymethylierungsprodukte von Hexylmethylamin, Hexyldimethylamin, Octyldimethylamin, Decyldimethylamin, Dodecylmethylamin, Dodecyldimethylamin, Dodecylethylmethylamin, C_{12/14}-Kokosalkyldimethylamin, Myristyldimethylamin, Cetyldimethylamin, Stearyldimethylamin, Stearylethylmethylamin, Oleyldimethylamin, C_{16/18}-Talgalkyldimethylamin sowie deren technische Gemische.

Weiterhin kommen auch Carboxyalkylierungsprodukte von Amidoaminen in Betracht, die sich durch die Formel 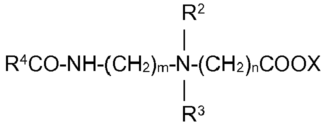 beschreiben lassen, in der R⁴CO für einen aliphatischen Acylrest mit 6 bis 22 Kohlenstoffatomen und 0 oder 1 bis 3 Doppelbindungen, m für Zahlen von 1 bis 3 steht und R², R³, n und X die oben angegebenen Bedeutungen haben. Typische Beispiele sind Umsetzungsprodukte von Fettsäuren mit 6 bis 22 Kohlen-stoffatomen, namentlich Capronsäure, Caprylsäure, Caprinsäure, Laurinsäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure so-wie deren technische Gemische, mit N,N-Dimethylaminoethylamin, N,N-Dimethylaminopropylamin, N,N-Diethylaminoethylamin und N,N-Diethylaminopropylamin, die mit Natriumchloracetat kondensiert werden. Bevorzugt ist der Einsatz eines Kondensationsproduktes von C_{8/18}-Kokosfettsäure-N,N-dime-thylaminopropylamid mit Natriumchloracetat.

Weiterhin kommen als geeignete Ausgangsstoffe für die im Sinne der Erfindung einzusetzenden Betaine auch Imidazoline in Betracht, die sich durch die Formel 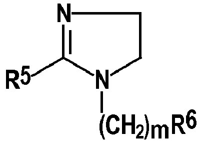 beschreiben lassen, in der R⁵ für einen Alkylrest mit 5 bis 21 Kohlenstoffatomen, R⁶ für eine Hydroxylgruppe, einen OCOR⁵- oder NHCOR⁵-Rest und m für 2 oder 3 steht. Auch bei diesen Substanzen handelt es sich um bekannte Stoffe, die beispielsweise durch cyclisierende Kondensation von 1 oder 2 Mol Fettsäure mit mehrwertigen Aminen, wie beispielsweise Aminoethylethanolamin (AEEA) oder Diethylentriamin erhalten werden können. Die entsprechenden Carboxyalkylierungsprodukte stellen Gemische unterschiedlicher offen-kettiger Betaine dar. Typische Beispiele sind Kondensationsprodukte der oben genannten Fettsäuren mit AEEA, vorzugsweise Imidazoline auf Basis von Laurinsäure oder wiederum C_{12/14}-Kokosfettsäure, die anschließend mit Natriumchloracetat betainisiert werden.

Bevorzugte erfindungsgemäße Reinigungsmittel sind dadurch gekennzeichnet, dass sie 0,5 bis 15 Gew.-%, vorzugsweise 1 bis 10 Gew.-%, weiter bevorzugt 1,5 bis 7,5 Gew.-%, noch weiter bevorzugt 2 bis 6 Gew.-% und insbesondere 2,5 bis 5 Gew.-% Tensid(e) aus der Gruppe der Betaine enthalten.

Die erfindungsgemäßen Reinigungsmittel enthalten als dritten Inhaltsstoff 0,1 bis 10 Gew.-% Betain (*N,N,N*-Trimethylammonioacetat bzw. *N,N,N*-Trimethylglycin). Vorzugsweise wird Betain sowohl innerhalb engerer Mengenbereiche als auch aus nativen Quellen eingesetzt.

Hier sind erfindungsgemäße kosmetische Reinigungsmittel bevorzugt, die 0,1 bis 10 Gew.-%, vorzugsweise 0,2 bis 9 Gew.-%, weiter bevorzugt 0,3 bis 8 Gew.-%, noch weiter bevorzugt 0,4 bis 7 Gew.-%, noch weiter bevorzugt 0,45 bis 6 Gew.-% und insbesondere 0,5 bis 5 Gew.-% natürliches Betain enthalten.

Als weiteren Inhaltsstoff können die erfindungsgemäßen Mittel mindestens ein natürliches, nicht chemisch modifiziertes Polymer enthalten. Solche auch als Biopolymere bezeichneten Substanzen stammen insbesondere aus der Gruppe der Polysaccharide. Besonders bevorzugt ist der Einsatz von Cellulose, Stärke, Guar Gum oder Xanthan.

"Nicht chemisch modifiziert" bedeutet, dass das natürliche Polymer keinen chemischen Reaktionen unterworfen wurde, um seine Eigenschaften zu beeinflussen. Physikalische Modifikationen hingegen sind möglich und z.B. bei Stärke und Xanthan geläufig (vorverkleisterte, gekochte, kaltquellende oder instantisierte Stärken, wärmebehandeltes Xanthan). Physikalisch modifizierte Stärken sind den nativen Stärken gleichgestellt, weil sie nur thermisch behandelt, d. h. gekocht sind.

Mit besonderem Vorzug enthalten die erfindungsgemäßen Mittel, wenn sie ein Biopolymer enthalten, 0,1 bis 5 Gew.-% Guaran (INCI-Bezeichnung Guar Gum).
Guaran, auch Guargummi genannt, ist ein Pflanzengummi. Die chemische Verbindung aus der Gruppe der Polysaccharide ist Hauptbestandteil von Guarkernmehl (oder kurz Guarmehl). Guaran besteht aus D-Mannopyranoseeinheiten, die über β--glycosidische Bindungen kettenartig miteinander verknüpft sind. Außerdem trägt jede zweite Mannopyranoseeinheit über eine -Bindung α-D-Galactopyranosyl-Reste.

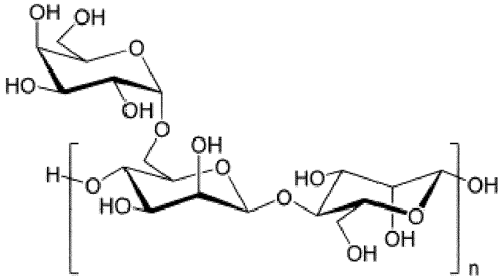

Vorzugsweise wird das Guar Gum in engeren Mengenbereichen eingesetzt. Hier sind bevorzugte erfindungsgemäße Reinigungsmittel dadurch gekennzeichnet, dass sie 0,2 bis 4 Gew.-%, vorzugsweise 0,3 bis 3,5 Gew.-%, weiter bevorzugt 0,4 bis 3 Gew.-%, noch weiter bevorzugt 0,45 bis 2,5 Gew.-% und insbesondere 0,5 bis 2 Gew.-% Guaran enthalten.

Die erfindungsgemäßen Mittel können, wenn sie ein Biopolymer enthalten, vorzugsweise 0,1 bis 5 Gew.-% Xanthan (INCI-Bezeichnung Xanthan Gum) enthalten. Xanthangummi ist ein natürlicher, nachwachsender Rohstoff und wird als ein anionisches Polysaccharid vom Bakterium *Xanthomonas campestris* ausgeschieden.
Das Molkülgewicht des eingesetzten Xanthangummis beträgt bevorzugt 2·10⁶ bis 20·10⁶ g/mol. Als Molekülbausteine enthält Xanthangummi D-Glucose, D-Mannose, D-Glucuronsäure, Acetat und Pyruvat in einem etwaigen molaren Verhältnis von 28 zu 30 zu 20 zu 17 zu 5,1 bis 6,3. Das Polymerrückgrat des Xanthangummis bildet sich aus einer Cellulose-Kette aus β-1,4-gebundenen Glucoseeinheiten. Xanthan enthält Struktureinheiten der folgenden Formel

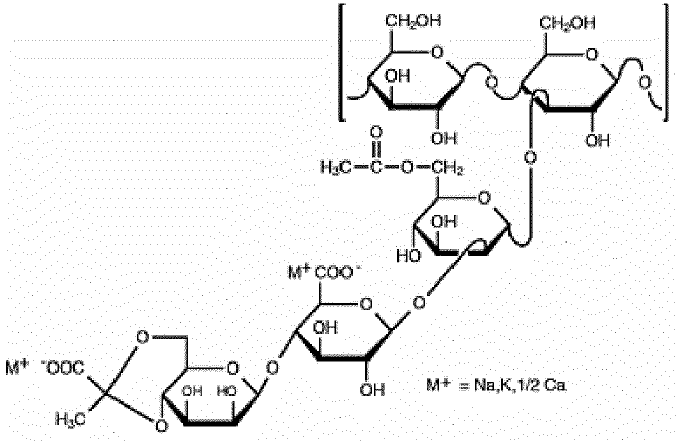

In den erfindungsgemäßen kosmetischen Mitteln kann auch gegebenenfalls wärmebehandeltes Xanthangummi eingesetzt werden.
In einer bevorzugten Ausführungsform enthält das erfindungsgemäße Mittel als Xanthangummi
- wärmebehandeltes Xanthangummi oder
- eine Mischung aus Xanthangummi und wärmebehandeltem Xanthangummi.

Bei Anwendung einer Mischung aus Xanthangummi und wärmebehandeltem Xanthangummi hat es sich als besonders wirksam herausgestellt, das Xanthangummi und das wärmebehandelte Xanthangummi in einem Gewichtsverhältnisbereich von 2 zu 1 bis 1 zu 20, insbesondere von 1 zu 2 bis 1 zu 10, einzusetzen.

Unter wärmebehandeltem Xanthangummi ist erfindungsgemäß Xanthangummi zu verstehen, welches Wärme von mindestens 40°C ausgesetzt wird. Das resultierende wärmebehandelte Xanthangummi weist eine verbesserte Dispergierbarkeit auf und lässt sich schneller in Wasser dispergieren, als Xanthangummi, das keiner Wärmebehandlung ausgesetzt wurde. Das bevorzugt geeignete wärmebehandelte Xanthangummi weist in einer 1 Gew.-%-igen wässrigen Lösung eine Viskosität von mindestens 25000 bis 40000 mPa.s (Brookfield DV-I Viskosimeter, Spindel #6 bei 23°C und 10 U/min) auf. Bevorzugt verwendbare, wärmebehandelte Xanthangummis liefern bei Herstellung einer 1 Gew.-%-igen, wässrigen Lösung einen pH-Wert von 4.0 bis 6.0 bei 23°C.

Das erfindungsgemäß bevorzugte wärmebehandelte Xanthangummi wurde durch temperieren von Xanthangummi bei einer Temperatur von mindestens 60°C, insbesondere von mindestens 100°C erhalten. Das Temperieren kann durch eine Vielzahl von bekannten Verfahren bewirkt werden, wie beispielsweise durch Ofen-, Fließbett-, Infrarot- oder Mikrowellenwärmebehandlung. Im Rahmen der vorgenannten Wärmebehandlungen ist es wiederum bevorzugt, wenn das Xanthangummi vor der Wärmebehandlung einen Wassergehalt von weniger als 25 Gew.-%, insbesondere von weniger als 8 Gew.-%, ganz besonders bevorzugt von weniger als 3 Gew.-%, aufweist. Es ist erfindungsgemäß weiterhin bevorzugt in dem erfindungsgemäß Mittel solches wärmebehandeltes Xanthangummi einzusetzen, das erhalten wurde, in dem Xanthangummi mit einem Wassergehalt von weniger als 25 Gew.-% bei einer Temperatur von mindestens 60°C (insbesondere von mindestens 100°C), für mindestens 30 Minuten wärmebehandelt wird. Es ist erfindungsgemäß besonders bevorzugt in dem erfindungsgemäß Mittel solches wärmebehandeltes Xanthangummi einzusetzen, in dem Xanthangummi mit einem Wassergehalt von weniger als 8 Gew.-% bei einer Temperatur von mindestens 60°C (insbesondere von mindestens 100°C), für mindestens 30 Minuten wärmebehandelt wird. Die bevorzugte Dauer der vorgenannten Wärmebehandlungen des Xanthangummis - insbesondere mit besagtem Wassergehalt - bei einer Temperatur von mindestens 60°C (insbesondere von mindestens 100°C) beträgt mindestens 1 Stunde. Die besonders bevorzugte Dauer der vorgenannten Wärmebehandlungen des Xanthangummis - insbesondere mit dem bevorzugten Wassergehalt - bei einer Temperatur von mindestens 60°C (insbesondere von mindestens 100°C) beträgt mindestens 2,5 Stunden.

Vorzugsweise wird das Xanthan Gum - unabhängig davon, ob es sich um wärmebehandeltes oder nicht wärmebehandeltes oder eine Mischung beider handelt - in engeren Mengenbereichen eingesetzt. Hier sind bevorzugte erfindungsgemäße Reinigungsmittel dadurch gekennzeichnet, dass sie 0,1 bis 10 Gew.-%, vorzugsweise 0,25 bis 9 Gew.-%, weiter bevorzugt 0,5 bis 8 Gew.-%, noch weiter bevorzugt 0,75 bis 7 Gew.-% und insbesondere 1 bis 6 Gew.-% Xanthan enthalten.

Die erfindungsgemäßen Mittel werden vorzugsweise wasserarm formuliert. Dies dient einerseits der Sicherstellung der festen Angebotsform, andererseits der Vermeidung unnötiger Produktvolumina, welche zu höherem Verpackungs- und Tramsportaufwand führen würden. Erfindungsgemäß bevorzugte kosmetische Reinigungsmittel sind dadurch gekennzeichnet, dass sie weniger als 25 Gew.-%, vorzugsweise weniger als 20 Gew.-%, weiter bevorzugt weniger als 15 Gew.-%, noch weiter bevorzugt weniger als 10 Gew.-% und insbesondere 0,1 bis 5 Gew.-% Wasser enthalten.

Mit besonderem Vorzug können die erfindungsgemäßen Mittel weitere Bestandteile enthalten, wobei insbesondere natürliche hautpflegende und rückfettende Substanzen geeignet sind. Besonders bevorzugt ist der Einsatz nativer Öle, so dass kosmetische Reinigungsmittel bevorzugt sind, die 0,1 bis 10 Gew.-%, vorzugsweise 0,25 bis 9 Gew.-%, weiter bevorzugt 0,5 bis 8 Gew.-%, noch weiter bevorzugt 0,75 bis 7 Gew.-% und insbesondere 1 bis 6 Gew.-% natürliche(s) Öl(e) enthalten.

Mit besonderem Vorzug enthalten die erfindungsgemäßen Mittel eines oder mehrere der nachstehend genannten Öle in einer Öl-Gesamtmenge wie vorstehend definiert: Açaíöl, Algenöl, Arganöl (aus den Früchten des Arganbaums), Avocadoöl (aus dem Fruchtfleisch der Avocado des Avocadobaums), Babaçuöl, Baumwollsamenöl (aus den Samen der Baumwollpflanze), Borretschöl oder Borretschsamenöl (aus den Samen der Borretschpflanze), Cupuagu-Butter, Cashew-Schalenöl, Distelöl (auch "Safloröl" genannt, aus den Samen der Färberdistel oder Carthamus), Erdnussöl (aus der Frucht der Erdnusspflanze), Haselnussöl (aus den Haselnüssen des Haselnussbusches), Hanföl (aus den Samen des Speisehanfs), Jatrophaöl (aus dem Samen der Jatropha curcas), Jojobaöl (eigentlich ein flüssiges Wachs; aus den Samen des Jojobastrauchs), Kamelieöl (aus den Samen der Camellia oleifera, Camellia sinensis oder Camellia japonica, Kakaobutter, Kokosöl (aus dem Samenfleisch der Kokosnuss, der Baumfrucht der Kokospalme), Kürbiskernöl (auch als *Kernöl* bezeichnet; aus den Samenkernen des Steirischen Ölkürbis), Leinöl (aus den reifen Leinsamen des Lein), Leindotteröl (aus den Samen des Leindotters, Familie der Kreuzblütengewächse), Macadamiaöl (aus den Nüssen des Macadamiabaums), Maiskeimöl (aus den Keimen von Mais), Mandelöl (aus den Mandeln des Mandelbaums), Mangobutter (aus Mangifera indica), Marillenkernöl bzw. Aprikosenkernöl (aus dem Aprikosenkern - also der Mandel des Aprikosensteins - der Aprikose bzw. Marille), Mohnöl (aus den Samenkörnern des Mohns), Nachtkerzenöl, Olivenöl (aus dem Fruchtfleisch und dem Kern der Olive, der Frucht des Olivenbaums, Palmöl (aus dem Fruchtfleisch der Palmfrucht, der Frucht der Ölpalme), Palmkernöl (aus den Kernen der Palmfrucht, der Frucht der Ölpalme), Papayaöl, Pistazienöl, Pekannussöl, Perillaöl aus den Samen der der Perilla-Pflanze (Shiso, Sesamblatt), Rapsöl (aus dem Samen von Raps, Familie der Kreuzblütengewächse), Reisöl, Rizinusöl (aus dem Samen des Wunderbaums), Sanddornöl (aus dem Fruchtfleisch der Sanddornbeere, der Frucht des Sanddornstrauches), Sanddornkernöl (aus den Kernen der Sanddornbeere, der Frucht des Sanddornstrauches), Senföl (aus den Samenkörnern des Schwarzen Senfs), Schwarzkümmelöl (aus den Samen der Fruchtkapsel der Schwarzkümmelpflanze), Sesamöl (aus den Samen der Sesampflanze), Sheabutter (aus den Samen des Sheanussbaums), Sojaöl (aus den Bohnen der Sojabohne), Sonnenblumenöl (aus den Kernen der Sonnenblume), Tungöl, Walnussöl (aus den Kernen der Nüsse des Walnussbaums), Wassermelonensamenöl, Traubenkernöl (aus den Kernen der Früchte (Weintraube) der Weinpflanze bzw. Weinrebe), Weizenkeimöl (aus den Keimen des Weizens), Zedernöl (aus dem Holz der Libanonzeder).

Die erfindungsgemäßen Mittel können weitere Inhaltsstoffe enthalten, wobei im Hinblick auf die Aufgabenstellung auf den Einsatz COSMOS-zertifizierter Rohstoffe geachtet werden sollte.

### Beispiele:

Folgende Zusammensetzung können beispielhaft hergestellt werden (alle Angaben in Gew.-%):

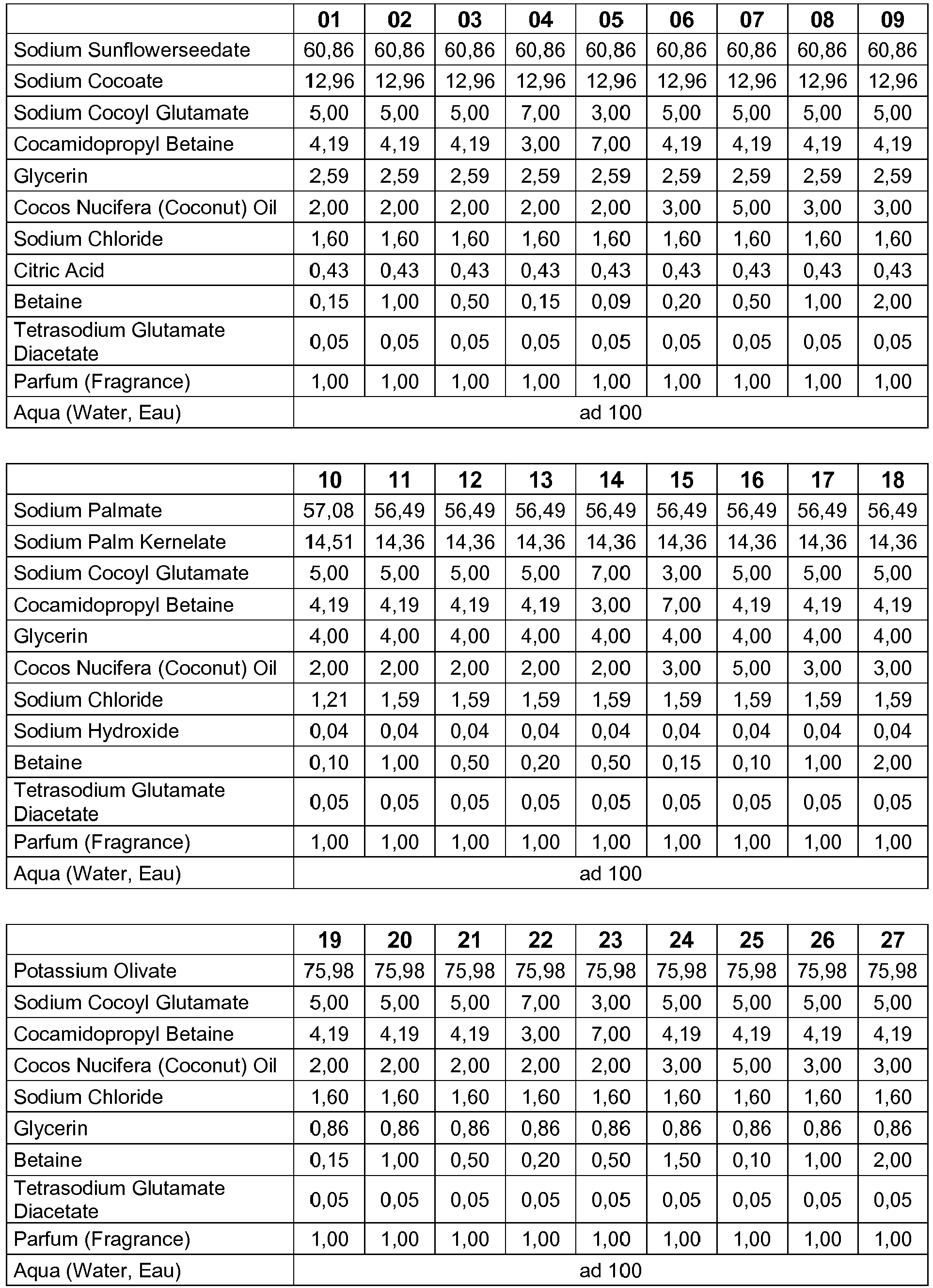

## Patentansprüche

1. Bei 25°C festes kosmetisches Reinigungsmittel, enthaltend, jeweils bezogen auf sein Gewicht,
a) 5 bis 90 Gew.-% Alkalisalze von Fettsäuren (Seifen);
b) 2 bis 50 Gew.-% Aclyglutamat(e);
c) 0,1 bis 10 Gew.-% Betain (*N,N,N*-Trimethylammonioacetat bzw. *N,N,N*-Trimethylglycin).

2. Kosmetisches Reinigungsmittel nach Anspruch 1, **dadurch gekennzeichnet, dass** es 10 bis 89 Gew.-%, vorzugsweise 20 bis 88 Gew.-%, weiter bevorzugt 30 bis 87 Gew.-%, noch weiter bevorzugt 40 bis 86 Gew.-% und insbesondere 50 bis 85 Gew.-% Alkalisalze von Fettsäuren (Seifen) enthält.

3. Kosmetisches Reinigungsmittel nach einem der vorangehenden Ansprüche, **dadurch gekennzeichnet, dass** es 10 bis 89 Gew.-%, vorzugsweise 20 bis 88 Gew.-%, weiter bevorzugt 30 bis 87 Gew.-%, noch weiter bevorzugt 40 bis 86 Gew.-% und insbesondere 50 bis 85 Gew.-% Natriumsalze von C₁₂₋₁₈-Fettsäuren enthält.

4. Kosmetisches Reinigungsmittel nach einem der vorangehenden Ansprüche, **dadurch gekennzeichnet, dass** es 3 bis 40 Gew.-%, vorzugsweise 4 bis 35 Gew.-%, weiter bevorzugt 5 bis 30 Gew.-%, noch weiter bevorzugt 6 bis 25 Gew.-% und insbesondere 7,5 bis 15 Gew.-% Acylglutamate der Formel **(I)** enthält 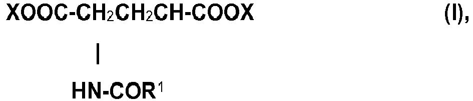 in der R¹CO für einen linearen oder verzweigten Acylrest mit 6 bis 22 Kohlenstoffatomen und 0 und/oder 1, 2 oder 3 Doppelbindungen und X für Wasserstoff, ein Alkali- und/oder Erdalkalimetall-, Ammonium-, Alkylammonium-, Alkanolammonium- oder Glucammoniumsteht.

5. Kosmetisches Reinigungsmittel nach Anspruch 4, **dadurch gekennzeichnet, dass** es ein Acylglutamat enthält, in dem X für Na und R¹CO für einen Acylrest steht, der von Kokosöl abgeleitet ist (INCI: Sodium Cocoyl Glutamate).

6. Kosmetisches Reinigungsmittel nach einem der vorangehenden Ansprüche, **dadurch gekennzeichnet, dass** es 0,1 bis 10 Gew.-%, vorzugsweise 0,2 bis 9 Gew.-%, weiter bevorzugt 0,3 bis 8 Gew.-%, noch weiter bevorzugt 0,4 bis 7 Gew.-%, noch weiter bevorzugt 0,45 bis 6 Gew.-% und insbesondere 0,5 bis 5 Gew.-% natürliches Betain enthält.

7. Kosmetisches Reinigungsmittel nach einem der vorangehenden Ansprüche, **dadurch gekennzeichnet, dass** es 0,1 bis 10 Gew.-%, vorzugsweise 0,25 bis 9 Gew.-%, weiter bevorzugt 0,5 bis 8 Gew.-%, noch weiter bevorzugt 0,75 bis 7 Gew.-% und insbesondere 1 bis 6 Gew.-% Xanthan enthält.

8. Kosmetisches Reinigungsmittel nach einem der vorangehenden Ansprüche, **dadurch gekennzeichnet, dass** es weniger als 25 Gew.-%, vorzugsweise weniger als 20 Gew.-%, weiter bevorzugt weniger als 15 Gew.-%, noch weiter bevorzugt weniger als 10 Gew.-% und insbesondere 0,1 bis 5 Gew.-% Wasser enthält.

9. Kosmetisches Reinigungsmittel nach einem der vorangehenden Ansprüche, **dadurch gekennzeichnet, dass** es 0,1 bis 10 Gew.-%, vorzugsweise 0,25 bis 9 Gew.-%, weiter bevorzugt 0,5 bis 8 Gew.-%, noch weiter bevorzugt 0,75 bis 7 Gew.-% und insbesondere 1 bis 6 Gew.-% natürliche(s) Öl(e) enthält.

10. Kosmetisches Reinigungsmittel nach einem der vorangehenden Ansprüche, **dadurch gekennzeichnet, dass** es - bezogen auf sein Gewicht - weniger als 1 Gew.-%, vorzugsweise weniger als 0,5 Gew.-%, weiter bevorzugt weniger als 0,5 Gew.-% und insbesondere weniger als 0,1 Gew.-% Sulfat-haltige(s) Tensid(e) enthält, wobei äußerst bevorzugte Mittel vollständig frei sind von sulfathaltigen Tensiden.
